# EUROPEAN PATENT SPECIFICATION

(11) **EP 1 264 584 B1**
(45) Date of publication and mention of the grant of the patent: **25.01.2006**
(21) Application number: 02011637.2
(22) Date of filing: 29.05.2002
(51) Int. Cl.: A61F 7/00, A61F 5/055

(54) **Collar device, particularly suitable for cervical thermotherapy**
Cervicalstütze, insbesondere für Cervicalthermotherapie
Minerve, particulièrement pour thermothérapie cervicale

(30) Priority: 01.06.2001 IT TV20010036
(43) Date of publication of application: 11.12.2002
(73) Proprietor: Barabino, Francesco, 31100 Treviso (IT)
(72) Inventor: Barabino, Francesco, 31100 Treviso (IT)
(74) Representative: Modiano, Micaela Nadia

(56) References cited:
- US-A- 5 211 623
- US-A- 5 295 949
- US-A- 5 507 793
- US-A- 5 785 670

## Description

The present invention relates to a collar device, particularly suitable for cervical thermotherapy.

Currently, one problem that troubles people, especially in winter periods or during cold and damp days, is that they suffer from torticollis, stiff neck or rheumatism in the shoulders or pain in the cervical vertebrae, caused by atmospheric conditions or by increasingly sedentary life.

To reduce these pains it is known to perform therapies such as localized massage performed by specialized physiotherapists or thermotherapy applications by using warming pads.

In both cases, however, high intervention costs are incurred, since it is necessary to perform several therapies of extended duration, and the pain persists in case of persistent bad weather.

In both cases, moreover, it is necessary to resort to treatment facilities, with consequent difficulty in obtaining timely appointments and with consequent delays in treatments which worsen the pain and therefore the physical discomfort, which negatively affects both private life and work.

Finally, such treatments require the user to physically go to the facilities dedicated to these treatments, consequently spending what might be a considerable amount of time for transfers and for the treatments and accordingly reducing one's leisure time and working time.

US-A-5 507 793 shows a soft collar device for cervical thermotherapy.

The aim of the present invention is to solve the noted problems by eliminating the drawbacks of the cited prior art, by providing a device that allows the user to have immediately available a remedy against torticollis, stiff neck or rheumatisms affecting the shoulders or pain affecting the cervical vertebrae.

Within this aim, an object of the present invention is to allow the user himself to take steps to achieve a remedy against torticollis, stiff neck or rheumatism of the shoulders or pain affecting the cervical vertebrae.

Another object is to provide a device that the user can use even in his own home.

Another object is to provide a device that allows the user to use it even during normal domestic or recreational or working activity.

Another object is to provide a device that can be used rapidly and simply by the user without any risk in use.

Another object of the present invention is to provide a device that has low costs and is structurally simple.

This aim and these and other objects that will become better apparent hereinafter are achieved by a collar device according to claim 1.

Further characteristics and advantages of the present invention will become better apparent from the following detailed description of a particular embodiment thereof, illustrated only by way of non-limitative example in the accompanying drawings, wherein:
Figure 1 is a partially sectional side perspective view of the collar device, particularly for cervical thermotherapy;
Figure 2 is a rear view of the collar device;
Figure 3 is a side view of the collar device, worn by a user;
Figure 4 is a front view of the collar device with the heating element removed.

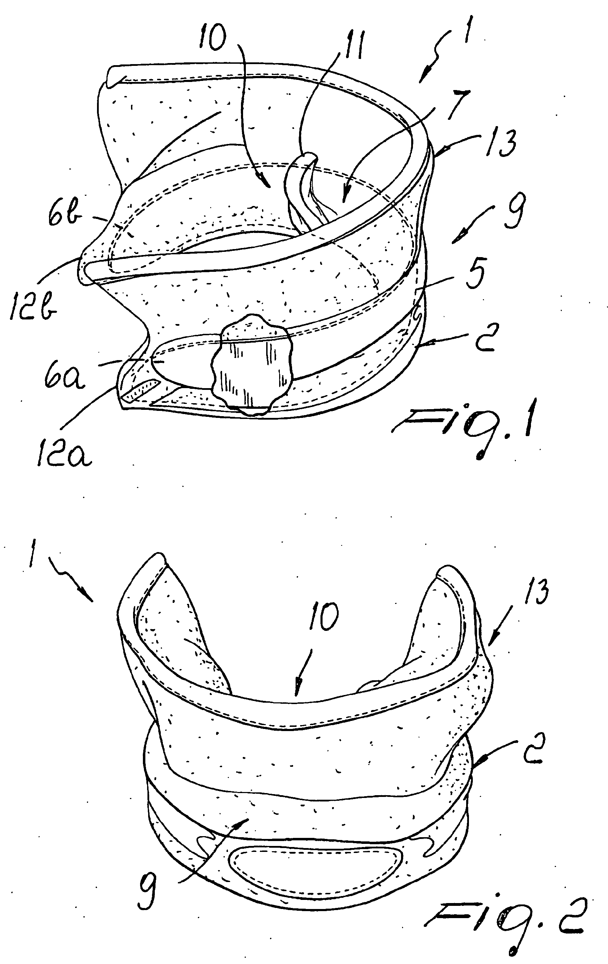

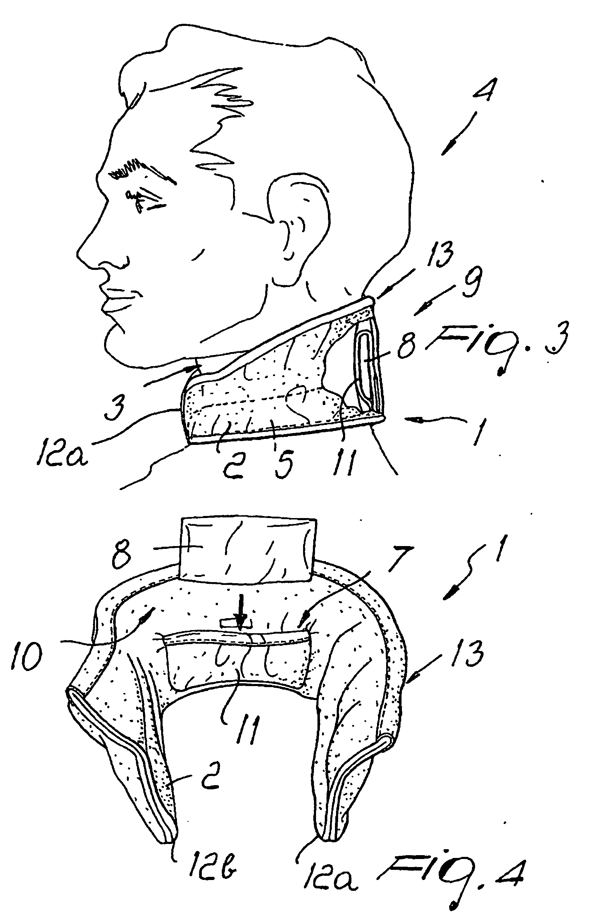

With reference to the figures, the reference numeral 1 designates a collar device particularly suitable for cervical thermotherapy.

Such collar device is constituted by a soft support 2, such as for example a fabric covering that is shaped so as to be able to wrap around the neck 3 of a user 4.

The support 2 contains at least one lamina 5 that can be shaped and is essentially U-shaped and advantageously concealed inside the support 2.

The lamina 5 advantageously has rounded ends 6a, 6b.

The lamina 5 further has a height that is approximately equal to the height of the support 2.

The lamina 5 is constituted by plastics, metallic or similar material that can be shaped and has characteristics that give it position memory.

This means that the material of which the lamina 5 is made can allow the user to impart thereto a chosen shape, so as to make the support 2 adhere more or less snugly to the neck of each individual user.

As regards use, it is sufficient for the user to grip the ends 6a and 6b of the lamina 5 contained within the support 2; in order to make the collar 1 adhere more snugly to the neck, it is sufficient to push forcefully the ends toward each other, crossing them and keeping them in this crossed position for a few seconds.

Vice versa, to make the collar device or collar 1 adhere less snugly to the neck, the user, after gripping the ends 6a and 6b of the lamina 5, merely has to forcefully widen them, moving them mutually apart.

The length of the lamina 5 is such that it does not wrap fully around the neck 3 of the user; this allows the collar to adhere the neck in an optimum manner without constricting the throat.

At the soft support 2 there is also at least one seat 7 for a removable element 8 that can be heated.

The seat 7 is preferably obtained at the region 9 of the nape of the neck 3, and is advantageously obtained by sewing, at the inner lateral surface 10 of the support 2, a band 11 of fabric that is open upward so as to define said seat or pocket for the heating element 8.

Such heating element can be constituted by a suitable sachet that contains materials suitable to produce an exothermic reaction upon contact with air.

As an alternative, the seat 7 can be obtained directly on the support 2 by forming thereon an opening that is for example slot-shaped and by placing thereat a containment pocket for the heating element 8.

The seat 7 can optionally be closed by means of a zip fastener or other known means such as press-studs or tapes or bands known under the trademark Velcro.

It is thus possible to have a pocket on the outside of the support 2 and an optional pocket inside the support.

The fabric of which the support 2 is made can be made of various materials, for example the synthetic material known as pile, or of natural material, such as wool or cotton or similar materials.

Inside the support 2, and in contact with the lamina 3, it is also possible to apply any kind of thermally insulating material or fabric that allows better and longer diffusion of the heat along the entire neck region surrounded by the lamina.

Moreover, the support 2 can have, proximate to its ends 6a and 6b, suitable engagement or closure means, such as press-studs, tapes or hook-and-loop bands, known by the trademark Velcro, which are suitable to keep the front ends 12a and 12b of the support 2 mutually in contact.

The support 2 can have, in an upward region, a raised flap 13 that acts as a protection against wind and cold air.

If the flap 13 is omitted, the present device has a reduced height and can be applied more discreetly, since in this case the collar can be concealed easily by using a high-neck sweater or any other item of clothing that can be worn by the user.

In order to activate the invention, it is sufficient for the user to first open a package that contains the element that can be heated, for example in the form of a sachet, which generates an exothermic reaction upon contact with air, and then insert said sachet at the appropriately provided seat 7.

The user then grips the collar at its front ends 12a and 12b, adapting them, as mentioned, to his own neck shape.

It is then sufficient to apply the collar so that the corresponding region provided with the seat for the heating element rests on the nape of the neck and release the front ends 12a and 12b of the support 2, which by way of the characteristics of the lamina 5 adheres to the nape and in general to the neck, allowing the heat to reach initially the entire neck and then, after a few minutes, also reach the shoulders and the back.

Thermotherapy thus begins and the beneficial effect of the heat can be perceived immediately.

This new invention therefore allows great advantages in the application of what is the most ancient and natural weapon against certain types of pain, i.e., heat.

The part of the body on which the present invention works is a very delicate one, i.e., the neck, and the use of the invention is recommended both when one simply wishes to be warmed and when one needs to alleviate or treat pain.

In the first case, the present invention acts like a modem neck warmer, which in addition to protecting from the cold releases a very pleasant warmth by means of the heating element that is used, which can be constituted by simple heating sachets that the invention is suitable to contain.

In the second case, the present invention acts as a comfortable, simple and useful portable warming pad, which requires no mains or battery power supply, since it utilizes the heat released by the heating sachets, which by way of the particular ingredients contained therein generate heat automatically and uninterruptedly for several hours upon contact with air.

The dimensions of said collar are so compact, and its application so practical, that it allows straightforward and very comfortable use.

Moreover, with the described collar it is possible to enjoy the benefits of cervical thermotherapy at any time of day: while riding the bus to work or even when working, or when doing housework or relaxing at home while listening to music.

The application of said collar is so comfortable that it causes no discomfort during use.

It has thus been observed that the invention has achieved the intended aim and objects, a collar having been provided which allows the user to have it available immediately when the pain starts by performing simple and quick operations that require no specific manual skills or training.

The collar can be used anywhere and in any moment of one's activity, both during daytime and while sleeping at night, without any danger since there is no electric power supply and the effect ceases simply when the chemical characteristics of the heating element are depleted.

Finally, the invention has low production costs; moreover, it is so comfortable to use that it can be employed both as a simple system for warming oneself and as an accessory in therapies for treating cervical problems, torticollis or simple rheumatisms.

The invention is of course susceptible of numerous modifications and variations, all of which are within the scope of the same appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A collar device, particularly suitable for cervical thermotherapy, comprising a soft support (2), which wraps around the neck (3) and has at least one seat (7) for a removable heating element (8), **characterized in that** it comprises at least one lamina (5) that can be shaped.

2. The collar device according to claim 1, **characterized in that** said soft support (2) is constituted by a covering made of fabric, which is shaped so that it can wrap around the neck (3) of a user.

3. The collar device according to claims 1 and 2, **characterized in that** said support (2) contains at least one lamina (5) that can be shaped and is essentially U-shaped and concealed inside said support (2).

4. The collar device according to claims 1 and 3, **characterized in that** said lamina (5), which has rounded ends (6a,6b), is approximately as high as said support (2).

5. The collar device according to claims 1, 3 and 4, **characterized in that** said lamina (5) is made of plastics, metallic or similar material that can be shaped, which has characteristics that give it position memory, said material allowing the user to give a chosen shape to said lamina (5), so as to make said support (2) adhere more or less snugly to the neck (3) of each individual user.

6. The collar device according to claims 1, 4 or 5, **characterized in that** the extension of said lamina (5) is such that it does not wrap fully around the user's neck (3), allowing the collar device (1) to adhere in an optimum manner to the neck (3) without constricting the throat.

7. The collar device according to claims 1 and 6, **characterized in that** at said soft support (2) said at least one seat (7) is provided, which accommodates a removable element (8) that can be heated, said at least one seat (7) being obtained preferably at a region (9) of the nape of the neck (3).

8. The collar device according to claims 1 and 7, **characterized in that** said at least one seat (7) is obtained by sewing, at the inner lateral surface (10) of said support (2), a band (11) of fabric that is open in an upward region so as to form said seat or pocket (7) for said heating element (8).

9. The collar device according to claims 1 and 8, **characterized in that** said heating element (8) is constituted by a suitable sachet which contains materials suitable to generate an exothermic reaction upon contact with air.

10. The collar device according to claims 1 and 7, **characterized in that** said at least one seat (7) is obtained directly on said support (2) by forming thereon an opening that is preferably slot-shaped and with which a pocket for containing said heating element (8) is associated.

11. The collar device according to claims 1 and 7, **characterized in that** said at least one pocket is external or internal to said support (2).

12. The collar device according to one or more of the preceding claims, **characterized in that** the fabric of which said support (2) is composed is constituted by synthetic material known as pile or by natural material such as wool or cotton.

13. The collar device according to one or more of the preceding claims, **characterized in that** a chosen type of thermally insulating fabric or material is applied inside said support (2) and in contact with said lamina (5) and allows better and longer diffusion of the heat over the entire region of the neck (3) surrounded by said lamina (5).

14. The collar device according to one or more of the preceding claims, **characterized in that** said support (2) has, proximate to the ends (6a,6b) of said lamina (5), suitable engagement or closure means such as press-studs, tapes, laces or hook-and-loop bands, which are suitable to keep the front ends (12a,12b) of said support mutually adjacent.

15. The collar device according to one or more of the preceding claims, **characterized in that** said support (2) has, in an upward region, a raised flap (13) that acts as a protection against wind and cold air.

16. A collar device according to claim 2 in combination with a heat-generating sachet (8), contained in said seat or pocket.

17. The collar device according to claim 15, **characterized in that** it has a reduced height achieved by the omission of said flap (13) so as to allow concealing of said collar device (1) with an item of clothing that can be worn by the user.

## Patentansprüche

1. Halsmanschettenvorrichtung, insbesondere geeignet für zervikale Wärmetherapie, die einen um den Hals (3) zu legenden weichen Träger (2) enthält, der mindestens eine Halterung (7) für ein abnehmbares Wärmeelement (8) aufweist,
**dadurch gekennzeichnet, dass** sie mindestens ein dünnes, formbares Flächenelement (5) enthält.

2. Halsmanschettenvorrichtung nach Anspruch 1,
**dadurch gekennzeichnet, dass** der weiche Träger (2) durch eine Gewebeabdeckung gebildet wird, die so geformt ist, dass sie um den Hals (3) des Benutzers gelegt werden kann.

3. Halsmanschettenvorrichtung nach den Ansprüchen 1 und 2,
**dadurch gekennzeichnet, dass** der Träger (2) mindestens ein dünnes Flächenelement (5) enthält, das formbar und im wesentlichen U-förmig ausgebildet und verdeckt im Träger (2) angeordnet ist.

4. Halsmanschettenvorrichtung nach den Ansprüchen 1 und 3,
**dadurch gekennzeichnet, dass** das dünne Flächenelement (5), das abgerundete Enden (6a, 6b) aufweist, etwa die gleiche Höhe hat wie der genannte Träger (2).

5. Halsmanschettenvorrichtung nach den Ansprüchen 1, 3 und 4
**dadurch gekennzeichnet, dass** das Flächenelement (5) aus Kunststoff, Metall oder einem ähnlichen verformbaren Material besteht, dessen Eigenschaften ihm eine Positionsgedächtnisfunktion verleihen, das es dem Benutzer erlaubt, dem Flächenelement (5) eine ausgewählte, den Träger (2) mehr oder weniger fest um den Hals (3) des jeweiligen Benutzers anordnende Form zu geben.

6. Halsmanschettenvorrichtung nach den Ansprüchen 1, 4 oder 5,
**dadurch gekennzeichnet, dass** die Länge des Flächenelementes (5) so gewählt ist, dass es den Hals (3) des Benutzers auf eine Weise nicht vollständig umschließt, dass die Halsmanschettenvorrichtung (1) in optimaler Weise am Hals (3) anliegt, ohne die Kehle zu beengen.

7. Halsmanschettenvorrichtung nach den Ansprüchen 1 und 6,
**dadurch gekennzeichnet, dass** an dem weichen Träger (2) die mindestens eine Halterung (7), die ein abnehmbares Wärmeelement (8) aufnimmt, vorzugsweise im Nackenbereich (9) des Halses (3) angeordnet ist.

8. Halsmanschettenvorrichtung nach den Ansprüchen 1 und 7,
**dadurch gekennzeichnet, dass** die mindestens eine Halterung (7) aus einem seitlich an die Innenfläche (10) des Trägers (2) derart angenähtem Gewebeband (11) besteht, dass ein oberer Bereich des Bandes (11) offen ist und die Halterung oder Tasche (7) für das Wärmeelement (8) bildet.

9. Halsmanschettenvorrichtung nach den Ansprüchen 1 und 8,
**dadurch gekennzeichnet, dass** das Wärmeelement (8) aus einem geeigneten Kissen besteht, das Materialien enthält, die bei Kontakt mit Luft eine exothermische Reaktion aufweisen können.

10. Halsmanschettenvorrichtung nach den Ansprüchen 1 und 7,
**dadurch gekennzeichnet, dass** die mindestens eine Halterung (7) unmittelbar am Träger (2) **dadurch** gebildet ist, dass am Träger eine vorzugsweise schlitzförmige Öffnung vorgesehen ist, mit der eine Tasche zur Aufnahme des Wärmeelementes (8) verbunden ist.

11. Halsmanschettenvorrichtung nach den Ansprüchen 1 und 7,
**dadurch gekennzeichnet, dass** die mindestens eine Tasche gegenüber dem Träger (2) außen oder innen angeordnet ist.

12. Halsmanschettenvorrichtung nach einem oder mehreren der vorhergehenden Ansprüche,
**dadurch gekennzeichnet, dass** der Träger (2) aus einem Synthetikmaterial mit Oberflächenflor wie Vlies oder aus Naturfasern wie Wolle oder Baumwolle besteht.

13. Halsmanschettenvorrichtung nach einem oder mehreren der vorhergehenden Ansprüche,
**dadurch gekennzeichnet, dass** innen im Träger (2) und im Kontakt mit dem Flächenelement (5) ein Wärme isolierendes Gewebe oder Material so angeordnet ist, dass eine bessere und längere Wärmediffusion über den gesamten vom Flächenelement umgebenen Hals (3) erfolgen kann.

14. Halsmanschettenvorrichtung nach einem oder mehreren der vorhergehenden Ansprüche,
**dadurch gekennzeichnet, dass** an dem Träger (2) im Bereich der Enden (6a, 6b) des Flächenelementes (5) geeignete Befestigungs- oder Verschlussmittel wie Druckknöpfe, Bänder oder Klettbänder vorgesehen sind, die geeignet sind, die vorderen Enden (12a, 12b) des Trägers aneinander zu halten.

15. Halsmanschettenvorrichtung nach einem oder mehreren der vorhergehenden Ansprüche,
**dadurch gekennzeichnet, dass** an dem Träger (2) in einem oberen Bereich ein erhöhter Randstreifen (13) als Schutz vor Wind und kalter Luft angeordnet ist.

16. Halsmanschettenvorrichtung nach Anspruch 2 in Kombination mit einem Wärme abgebenden Kissen (8), das in der genannten Halterung oder Tasche angeordnet ist.

17. Halsmanschettenvorrichtung nach Anspruch 15,
**dadurch gekennzeichnet, dass** sie durch Weglassen des erhöhten Randstreifens (13) eine so verringerte Höhe aufweist, dass die Halsmanschettenvorrichtung (1) durch ein vom Benutzer getragenes Kleidungsstück verdeckt werden kann.

## Revendications

1. Minerve, particulièrement pour la thermothérapie cervicale, comprenant un support souple (2) qui s'enroule autour du cou (3) et qui comporte au moins un support (7) pour un élément chauffant amovible (8), **caractérisée en ce qu'**elle comprend au moins une lamina (5) qui peut être mise en forme.

2. Minerve selon la revendication 1, **caractérisée en ce que** ledit support souple (2) se compose d'un recouvrement réalisé dans du tissu, qui est mis en forme de manière à pouvoir s'enrouler autour du cou (3) d'un utilisateur.

3. Minerve selon les revendications 1 et 2, **caractérisée en ce que** ledit support (2) contient au moins une lamina (5) qui peut être mise en forme, a sensiblement la forme d'un U et est cachée à l'intérieur dudit support (2).

4. Minerve selon les revendications 1 et 3, **caractérisée en ce que** ladite lamina (5), qui présente des extrémités arrondies (6a, 6b), a à peu près la même hauteur que ledit support (2).

5. Minerve selon les revendications 1, 3 et 4, **caractérisée en ce que** ladite lamina (5) est réalisée dans du plastique, un matériau métallique ou similaire qui peut être mis en forme, qui présente des caractéristiques qui lui confèrent une mémoire de position, ledit matériau permettant à l'utilisateur de donner une forme choisie à ladite lamina (5) de manière à faire coller ledit support (2) de manière plus ou moins serrée au cou (3) de chaque utilisateur individuel.

6. Minerve selon la revendication 1, 4 ou 5, **caractérisée en ce que** l'extension de ladite lamina (5) est telle qu'elle ne s'enroule pas complètement autour du cou (3) de l'utilisateur, ce qui permet à la minerve (1) de coller de manière optimale au cou (3) sans serrer la gorge.

7. Minerve selon les revendications 1 et 6, **caractérisée en ce que**, au niveau dudit support souple (2), est prévu ledit au moins un support (7), qui accueille un élément amovible (8) qui peut être chauffé, ledit au moins un support (7) étant obtenu de préférence au niveau d'une zone (9) de la nuque (3).

8. Minerve selon les revendications 1 et 7, **caractérisée en ce que** ledit au moins un support (7) est obtenu en cousant, au niveau de la surface latérale intérieure (10) dudit support (2), une bande (11) de tissu qui est ouverte dans une zone orientée vers le haut de manière à former ledit support ou ladite poche (7) pour ledit élément chauffant (8).

9. Minerve selon les revendications 1 et 8, **caractérisée en ce que** ledit élément chauffant (8) se compose d'un paquet approprié qui contient des matériaux appropriés pour générer une réaction exothermique au moment de son entrée en contact avec l'air.

10. Minerve selon les revendications 1 et 7, **caractérisée en ce que** ledit au moins un support (7) est obtenu directement sur ledit support (2) en formant sur celui-ci une ouverture qui est de préférence en forme de fente et à laquelle est associée une poche destinée à contenir ledit élément chauffant (8).

11. Minerve selon les revendications 1 et 7, **caractérisée en ce que** ladite au moins une poche est externe ou interne audit support (2).

12. Minerve selon une ou plusieurs des revendications précédentes, **caractérisée en ce que** le tissu dont se compose ledit support (2) se compose d'une matière synthétique connue sous le nom de feutre ou d'un matériau naturel comme la laine ou le coton.

13. Minerve selon une ou plusieurs des revendications précédentes, **caractérisée en ce qu'**un type choisi de tissu ou de matériau thermiquement isolant est appliqué à l'intérieur dudit support (2) et vient en contact avec ladite lamina (5) et permet de diffuser la chaleur mieux et plus longtemps sur toute la zone du cou (3) entourée par ladite lamina (5).

14. Minerve selon une ou plusieurs des revendications précédentes, **caractérisée en ce que** ledit support (2) comporte, à proximité des extrémités (6a, 6b) de ladite lamina (5), des moyens de mise en prise ou de fermeture appropriés comme des boutons-pression, des rubans, des lacets ou des bandes Velcro, qui sont appropriés pour maintenir les extrémités avant (12a, 12b) dudit support adjacentes l'une à l'autre.

15. Minerve selon une ou plusieurs des revendications précédentes, **caractérisée en ce que** ledit support (2) comporte, dans une zone orientée vers le haut, un rabat élevé (13) qui sert de protection contre le vent et l'air froid.

16. Minerve selon la revendication 2, en combinaison avec un paquet générateur de chaleur (8) contenu dans ledit support ou ladite poche.

17. Minerve selon la revendication 15, **caractérisée en ce que** sa hauteur réduite est obtenue en retirant ledit rabat (13) de manière à permettre de cacher ladite minerve (1) avec un vêtement que l'utilisateur peut porter.
